# EUROPEAN PATENT SPECIFICATION

(11) **EP 2 310 876 B1**
(45) Date of publication and mention of the grant of the patent: **19.03.2014**
(21) Application number: 09788144.5
(22) Date of filing: 27.07.2009
(51) Int. Cl.: G01T 1/164

(54) **GAMMA RADIATION IMAGING APPARATUS**
GAMMASTRAHLUNGS-BILDGEBUNGSVORRICHTUNG
APPAREIL D'IMAGERIE PAR RAYONNEMENT GAMMA

(30) Priority: 29.07.2008 NL 2001858
(43) Date of publication of application: 20.04.2011
(73) Proprietor: Milabs B.V., 3584 CX Utrecht (NL)
(72) Inventor: BEEKMAN, Frederik, Johannes, NL-3584 CG Utrecht (NL)
(74) Representative: Brookhuis, Hendrik Jan Arnold
(86) International application number: PCT/NL2009/000155
(87) International publication number: WO 2010/014001

(56) References cited:
- EP-A2- 1 237 013
- EP-A2- 1 336 869
- NL-C1- 1 030 168
- US-A1- 2008 001 088
- "Simple surface markers for aligning single photon emission computed tomography (SPECT) and magnetic resonance (MRI) images - Mantosh Dewan, Steven Falen, Nikolaus Szeverenyi, Prakash Masand, John Tanquary, Minda Lynch, F. Deaver Thomas SUNY Health Science Center at Syracuse, Syracuse, NY 13210" BIOLOGICAL PSYCHIATRY, ELSEVIER SCIENCE, NEW YORK, NY, US LNKD- DOI:10.1016/0006-3223(92)90499-P, vol. 31, no. 5, 1 March 1992 (1992-03-01), page 123, XP024252141 ISSN: 0006-3223 [retrieved on 1992-03-01]

## Description

The present invention relates to a gamma radiation imaging apparatus, comprising an object positioning device, defining an imaging space for the object to be imaged, and a gamma camera positioned to image a volume in said imaging space, wherein the object positioning device comprises two plates, with the imaging space there between, wherein at least one of the plates is movable in a direction substantially towards the other plate, and wherein the plates are arranged to contact the object when positioned between said plates.

Such apparatus are known in the art. For example, document US2007/0096027 discloses an apparatus with a first and second gamma camera configured to compress an object there between, optionally with a collimator, without further details being given.

The known apparatus suffer from the drawback that a relatively large fraction of the images are insufficiently accurate, for example false positive detections of tumors. This leads to an undesirably and unnecessarily high number of biopsies, tumor surgeries and breast amputations and also to a higher than necessary number of images being taken, which can cause discomfort or even pain to the patient, in particular to women of whom the breast is being imaged. In addition it can lead to undetected small tumors or parts of tumors in which case no, or insufficient amounts of, tumor tissue will be removed by a surgeon.

It is an object of the present invention to provide apparatus of the kind indicated, in which the accuracy in terms of resolution and noise properties is improved, thus providing more reliable imaging.

This object is achieved with an apparatus according to claim 1.

This apparatus according to claim 1 makes optimum use of the very high resolving power that is attainable with such a set-up with a pinhole collimator. In particular, this apparatus uses the strategy to bring pinhole(s) very close to the relevant tissue. Note that sufficient angular information is obtainable by moving the collimator with respect to the object, i.e. in practice with respect to the plates. Although the information is achieved from different parts of the detector, and will not completely comprise 180° information, the increased resolution outweighs the arithmetic complexity and the chance of defects due to angularly incomplete data.

Note that "movable in a plane" is intended to mean that the collimator is able to move in at least two different directions in said plane. A preferred movement is a zig-zag movement, in which the collimator moves up and down in an x-y grid, from one side to the other. An alternative movement is a spiral movement, in which the collimator starts in the middle and moves in an outwardly directed, circular movement.

The apparatus according to the invention is preferably embodied as a breast imaging apparatus for imaging of woman's breast, e.g. to detect tumors or associated effects within the breast, but can also be embodied for imaging other body parts, such as limbs, e.g. for detecting skin cancer, or e.g. the lymph nodes.

Preferably during imaging the breast is compressed between the plates, the apparatus comprising means to cause said compression.

Note that in the art, scanning apparatus are known in which the detector rotates around the object to be imaged, such as a breast. In such a case, it is logical not to compress the breast, since then it will retain its more or less rounded shape, ensuring on average the lowest distance between scanner and breast. However, this also means that the distance between the object, in particular parts of the breast, and the detector can not become very small. Compressing the breast does not work here, since then there is always a part at the ends with a larger distance then uncompressed, which larger distance determines the radius for rotating, which becomes unfavorable with respect to the uncompressed situation. In addition in such devices it is very hard to minimize the distance to the breast since the optimal orbit of the gamma camera depends on shape of the breast.

Contrarily, with the apparatus of the present invention the planes between which the breast is contained are exactly known and the distance between pinholes and breast lesion may be made very small, down to the thickness of one of the plates, and of course the collimator and the desired distance between the collimator and the detector. The apparatus of the present invention uses this close distance by allowing to scan parallel to the very plate that may contact the breast, or even compress the breast to a certain degree. This small distance is a great advantage. In embodiments, the collimator is substantially contiguous to one of the plates. Herein, this is understood as the distance between the relevant plate and the collimator being smaller than 3 cm, in particular substantially zero, i.e. contact between the plate and the collimator, apart from a little play to allow the movement.

Furthermore, it is not necessary for the detector to be parallel to the collimator. Although this might lead to a somewhat distorted image, this distortion is relatively easily corrected, while this allows a favorable positioning of the pinhole(s) of the collimator and of the detector with respect to the object. The central line of the pinhole will often be at a sharp angle with respect to the plate to allow full imaging.

Preferably, the detector is a position sensitive detector. Such a detector gives information about in which part of the detector the gamma photon has interacted.

EP 1237013, from which claim 1 is delimited, discloses a gamma camera apparatus which comprises a gamma camera for detecting gamma rays radiated from an examinee lying on a bed and an image reconstructing unit for reconstructing a three-dimensional image of the examinee based on gamma rays detected by the gamma camera. The gamma camera has a main camera unit, a plate supported on the main camera unit by a pair of guide bars for keeping the main camera unit spaced a certain distance from the examinee, and a collimator disposed between the main camera unit and the plate. The collimator can be displaced between the main camera unit and the plate by a screw. The collimator has a number of pinhole apertures defined therein. A high-resolution estimated projected image of the examinee can be obtained by adjusting the position of the collimator. The collimator is not movable with respect to the bed and the plate in a plane substantially parallel to the bed or the plate.

In a preferred embodiment the imaging apparatus comprises a further or second gamma camera which comprises a collimator with at least one pinhole, and a gamma sensitive detector arranged to receive images from the collimator, wherein the collimator is arranged in a plane substantially parallel to one of the plates and is preferably movable in said plane, and wherein the apparatus further preferably comprises a collimator mover means arranged to controllably move the collimator in said plane, and wherein the gamma camera and the further gamma camera are arranged on the opposite sides of the imaging space.

In a preferred embodiment the imaging apparatus comprises a frame that supports the plates in a mobile manner so as to be movable in a direction substantially towards one another allowing to compress the object, e.g. a woman's breast, between the plates.

Preferably the plates are parallel to one another and movable perpendicular to their plane.

In general compression will cause the object to remain in place with respect to the plates and the camera, so enhancing the quality of the image. Also compression, at least in case of a breast, makes the object to be imaged thinner, and the obtained images more unambiguous. Amongst the advantages of breast compression are a larger contact area, and a larger chance that tumors are present on the surface. The closer the pinhole is to the object, the larger the sensitivity (improved detection) and the spatial resolution, this will increase the detected fraction of emitted gamma-photons.

In the following, the object to be imaged will be taken to be a woman's breast, although other objects, in particular those which are compressible, are also possible. In principle, contacting the breast with the plates also brings great advantages as to resolution.

In embodiments, the apparatus comprises even a third or fourth gamma camera, comprising a collimator and a gamma sensitive detector. The first gamma camera and second gamma camera are preferably arranged on opposite sides of the imaging space. It is possible to provide the object positioning device with a third plate, extending essentially perpendicular to and between the two plates, further defining the imaging space. The collimator of a third gamma camera may be arranged in a plane substantially parallel to this third plate. As such, this third camera may be very useful to provide information about the third dimension, i.e. depth.

In certain embodiments, the two or more gamma cameras are of a similar or even identical type, which makes evaluation of their images easier and production more efficient.

In a preferred embodiment, the movements of gamma camera(s) are such that it allows to reconstruct the images to create a SPECT image. In particular, the gamma camera(s) is/are arranged to image very high energy photons (e.g. 511 keV) that can normally only be detected by coincidence PET systems. Imaging in combination with a collimator gives a lower sensitivity than coincidence PET cameras, but an even increased resolution when pinhole openings are narrow, which also allows imaging of very high energy photons. It is however also possible to provide two different gamma cameras, such as with different detectors, that have different detection efficiencies, e.g. for different energy, or that have different pinholes and so on.

In practice, the collimator mover means are arranged for controllably moving only the collimator in a plane parallel to the respective plates, while the detector remains generally stationary. The advantage is that the collimator has a very limited weight, whereas the detector usually is very heavy. This may e.g. be embodied such that the collimator mover means can be moved along two orthogonal axes, or along a regular scanning path, a completely random path and so on. The collimator mover means may be computer-controlled.

In a possible embodiment the imaging apparatus is embodied such that the plates can assume a non-parallel position with respect to one another.

In a possible embodiment an imaging apparatus for breast imaging is adapted to position the plates, preferably parallel plates, at an inclined orientation with the higher end of the plates near or at the woman's armpit, so as to be able to image tissue near said armpit as well.

When the plates assume a non-parallel orientation the moving of the collimator moving means may be adapted to the angle, e.g. by scanning during a correspondingly longer time at a location where the tissue thickness is larger. Preferably, the angle between the plates is adjustable. Note that the collimator may thus also be positionable, i.e. under that adjustable angle.

In a possible embodiment the imaging apparatus is provided with contour detection means, e.g. an optical detection means, to detect the contour of the object to be imaged with the gamma camera while being held, preferably compressed, between the plates. Said contour detection means are different from the gamma camera and preferably being operational before the gamma camera is active. Preferably contour information obtained with said means is fed to the collimator mover means in order to adapted the path of motion of the collimator to the actual contour of the object. This e.g. allows for a far shorter imaging time for a small woman's breast compared to a large breast.

In a possible embodiment the imaging apparatus is provided with an automatic analyzer of a digital image obtained with a gamma camera, e.g. the automatic analyzer being a computer with suitable graphic image analysis software, the analyzer including a library of items, e.g. stored on the basis of actual surgery. In a possible embodiment the imaging apparatus is adapted to change the speed and/or path of the collimator in its plane based on commands from the automatic analyzer. For instance the library may contain graphic images of tumors; as soon as the analyzer detects (a portion) of a tumor, the collimator may be moved at a lower speed (for increased accuracy) and/or reversed over some distance to repeat said section of the path at a lower speed.

In another embodiment the imaging apparatus is embodied to make use of a selected one of multiple different pinhole systems, each pinhole system including one or more pinholes, the selection e.g. being based on input from an operator of the apparatus or based on the automatic analyzer mentioned above. For instance when the automatic analyzer detects an area that may require an improved quality image, the apparatus may change from one pinhole system to another operative pinhole system and then re-image said area. For example a single collimator member may include multiple pinhole systems, the mover means being adapted to bring a selected pinhole system in operative position with respect to the detector, whereas other pinhole system are at an inoperative location.

In an alternative embodiment, the gamma camera and the collimator are movable as a whole or unit in a plane substantially parallel to the plate, and the collimator mover means is arranged to controllably move the unit in said plane. One could say that in this case the detector, and possibly other parts of the gamma camera, are coupled to the collimator, and they are moved together with the collimator. By moving the camera or cameras as a whole, i.e. keeping the distance between detector and collimator fixed, calculations with respect to the obtained images are relatively easy. It is however also possible to arrange the collimator and detector for one or more gamma cameras such that the distance between them is adjustable, enabling a zoom effect.

The moving of the collimator(s), or gamma camera(s) as a whole, preferably is along a predetermined path, such as a zigzag path for scanning the whole breast. The collimator mover means may be arranged accordingly, such as by providing an accordingly programmed computer.

In embodiments, the apparatus comprises a plurality of different pinholes. The different pinholes could be provided on one collimator, or on different collimators.

Herein, "pinhole" comprises the concept of a hole in a wall of a radiation opaque material, while there are cones leading to and from the pinhole. These cones, that may have a (preferably) circular, elliptical, square etc. cross-section, determine the opening angle of the pinhole, while the pinhole itself largely determines the sensitivity, through its cross-sectional area. In this respect, holes in e.g. parallel hole collimators, which are nothing more than channels in the collimator plate, are not treated as pinholes in this application. It is conceivable that the collimator comprises only a single pinhole. This single pinhole is then arranged in a plane substantially parallel to one of the plates and is movable in said plane.

In the present application, providing different pinholes gives the possibility to adapt e.g. the sensitivity. By moving the collimator, such different pinholes may be positioned as desired with respect to the breast, for example with respect to a suspect part of the breast.

In particular, at least part of the plurality of pinholes are focused towards a focus volume. This means that multiple pinholes "look" at the same volume, the focus volume, and image same onto the detector. This greatly increases the sensitivity and angular information, and hence the resolution. In this respect, focusing is to be understood as focusing in two dimensions, i.e. the pinholes are distributed in a plane, instead of along a line, while the central lines of the respective pinholes in such a plurality point substantially to a single point. The cones of directions for radiation that is able to penetrate then all overlap in a small, mostly substantially rounded focus volume around said point.

In special embodiments, a first plurality of pinholes is focused towards a first focus volume, and a second plurality of pinholes is focused towards a second focus volume. In principle, the first and second volume do not overlap, although this is not necessary. What is important is that it allows differences in the pinholes and the focus volume. For example, the first focus volume could be larger than the second. Thus, the second plurality of pinholes images a smaller volume. For a similar number of pinholes, this thus increases relative sensitivity at the cost of overview.

In particular, a first plurality of pinholes has a first opening angle and a second plurality of pinholes has a second opening angle, that is smaller than the first opening angle. This allows e.g. the use of higher energy gamma tracers, as a smaller opening angle is generally "harder" to higher energy radiation. Sensitivity may be increased or compensated by increasing the number of pinholes. Alternatively, it is possible to use so-called cluster pinholes.

In a possible embodiment, the first plurality of pinholes is provided in the first collimator and the second plurality is provided in a second collimator. Such "dedicated" collimators allow a very flexible approach to imaging, in that e.g. first the breast is scanned coarsely but quickly with the collimator with large opening angle pinholes. When the image has been evaluated, the gamma camera with the collimator with small opening angle pinholes is used for a finer investigation. In the latter, it could be advantageous to allow a larger distance between pinholes and detector to get an enlarged picture with a higher resolution.

In a possible embodiment the imaging apparatus includes a collimator having multiple sets of one or more pinholes, the one or more pinholes forming one set being different from the pinholes forming another set. In a possible embodiment the imaging device includes a suitable indexing device for the collimator, allowing a selected set of pinholes to be in an operational position, whereas the one or more other sets of pinholes are at an non-operation position, e.g. the collimator being rotatable about an axis at right angles to a main plane of the collimator by a suitable indexing device. It is conceivable that the collimator comprises multiple sets of pinholes, revolver-like ordered to be interchangeable as desired.

In embodiments, the apparatus is arranged to image the object to be imaged in a fast mode, wherein only a collimator is moved in the plane, or all collimators are moved. This allows a very fast scan, because only the collimator has to be displaced and not the whole camera, as is the case in the prior art. A collimator is of course much lighter than the collimator plus the rest of a gamma camera, but since the present invention also has a very small object-to-pinhole distance, the sensitivity can be very high, all of which accounts for the high possible scan speeds.

A particular advantage of these embodiments is that they allow the recording of dynamic images. In particular, tracers like 99mTc-MIBI can be applied to e.g. suspect or tumor tissue, and then dynamically imaged to study the changes to the concentration of the tracer in the tissue. This is a helpful tool in studying phenomena like tumor response to chemotherapy. Note however, that the apparatus of the present invention may also be arranged to image the object to be imaged in a fast mode by moving a gamma camera (or the gamma cameras) as a whole. In each case, it is possible to provide dynamic images, or time-dependent images of the object.

Preferably, at least one set of one or more pinholes with an opening top angle of at most 40°, and preferably at most 25°, is provided, which give good, to excellent, imaging properties for high energy photons.

In some embodiments, at least one pinhole comprises a cross-slit pinhole. This is meant to indicate a pinhole that does not consist of a simple hole in the collimator plate, with cones leading to and from it, but the combination of two crossed, i.e. non-parallel, slits in two parallel collimator plates. This also has the effect of a pinhole. Note that the slits in the collimator plates should have a profile of a dual cone like in an ordinary pinhole, to ensure an opening angle etc.

In embodiments, at least one cross-slit pinhole comprises adjustable crossed slits. This allows variation of e.g. the width of the pinholes, and thus the sensitivity, by moving of the two collimator plates with respect to each other. It is also possible to shift the mutual position of the pinholes and so on.

In a preferred embodiment the imaging apparatus further comprises a marker system, that is arranged to mark the object that is to be imaged. More preferably, the marker system is adapted to mark the object while the object is positioned, preferably compressed, between the plates of the imaging apparatus, e.g. before, during and/or after the actual imaging takes place. This e.g. allows more reliable re-imaging of the object in a subsequent imaging, either with the same apparatus or with a different apparatus, such as an X-ray camera. The marking can be done with any desired kind of marker, but preferably relates to a visible marker for ease of use, e.g. using an ink. It is also conceivable to use an ink that is only visible in UV-light. Another alternative is to use a type of visible or non-visible ink that has magnetic properties, such that it is detectable in an MRI-system.

In embodiments, the marker system comprises a plurality of channels in or on at least one of the plates and having dispensing ports at the side of the imaging space, as well as a marker substance dispenser, arranged to controllably dispense marker substance through at least one of the channels and associated ports.

With a marker system, the physician or other operator of the apparatus is e.g. able to indicate the position of the breast in the apparatus, in particular with respect to the plates. The position of the collimator(s) or camera(s) as a whole are also known, with the help of the collimator mover means. Then, by registering one or more marked positions on the breast in a subsequent imaging, it can be ensured with a high degree of accuracy that the breast is positioned in the same way as with the previous imaging.

In another embodiment, the marker system is arranged to mark on the breast, or other object, the reconstructed image, or at least a selected part of said image. Preferably the marker system is associated with each of the plates, so that at least a selected part of the reconstructed image of the object may be marked on two sides of the object. This may provide valuable information to a surgeon, radiotherapist etc., by locating suspect or tumor tissue or other relevant spots.

In an exemplary embodiment a person, e.g. a radiotherapist, may study the reconstructed image and then select one or more items of interest in said image, e.g. using suitable graphic image processing software in conjunction with a digital version of the reconstructed image, e.g. that allows to select an item (such as a suspect region in the object) and then automatically create a contour line or other graphical indicator thereof. The person may then operate the marker system so that the contour line or other graphical indicator of said selected item is actually marked on the object. In another embodiment the reconstructed image in digital format may be automatically analyzed by a suitably programmed computer on a basis of a library of items, e.g. based on results obtained from actual surgery or other treatment. The automatically obtained analysis, may be represented e.g. in graphical form, e.g. by placing suitable indicators and/or contour lines in the reconstructed image. The analysis results may also be fed to the marker system, e.g. with intermediate screening by a person, e.g. a surgeon, to be marked on the object.

The marker system can also be adapted to mark on the object information about depth of the item of interest, when such depth information - as is preferred - is obtained via the imaging of the object.

The marking can be used when introducing wires or the like to indicate suspect or tumor tissue, e.g. with a plate with a mesh of channels through which the wire(s) may be inserted into the breast. In embodiments, the marker is gamma-radioactive, allowing the marker to be seen and used in a subsequent scan/image.

Preferably the actual distance between the plates and/or orientation of the plates, determining the degree of compression of the breast and/or position with respect to the breast, is also controlled, the imaging apparatus comprising a controlled displacement device for moving said one or more plates, e.g. with electric motors. Preferably said distance and/or orientation is memorized in a memory of the apparatus, so that the actual compression and/or orientation can be taken into account for later purposes, e.g. evaluation of the image obtained, repetition of the imaging procedure, etc.

The invention also relates to a method of gamma imaging a breast, using an apparatus according to the present invention. In the method, a breast is brought in the imaging space, and is imaged, wherein the collimator, or the whole gamma camera - less preferred -, is moved in a plane parallel to the plate. Since the distance between the pinhole(s) and the breast is small, favorable imaging properties (high sensitivity and resolution) can be obtained.

In particular when focused pinholes are used, the total breast may be scanned by moving the focus volume through the breast. As mentioned above, fast positioning sequences of the pinholes make it possible to obtain movies of tracer dynamics of the entire breast.

In embodiments of the method, using an apparatus that comprises a marker system according to the invention, the method comprises the steps of adjusting the plates to a desired degree of compressing the breast, recording distance d between the plates, imaging the breast, creating a plurality of marks on the breast in known positions with respect to the plate by means of the marker system, removing the apparatus from the breast, and performing a second imaging, wherein the second imaging comprises registering the marks on the breast with respect to said known positions on the plate, possibly adjusting the plates to said distance d, and imaging the breast.

This allows a very reliable way of registering the subsequent images with the first images. In particular, the type of imaging may be different the second time, such as X-ray imaging, or gamma imaging with a different set of pinholes. Also, it is possible to simply re-image the breast with the same settings, but after some time, e.g. after a biopsy has been performed and the sample been examined.

In a possible embodiment the frame having the two plates is separable from the gamma radiation imaging apparatus. For instance, when a second imaging apparatus is used to make an image of the object, e.g. based on a different imaging technique, e.g. based on X-ray, the frame with the plates can remain on the breast in the same position and then introduced into said second imaging device that is adapted to make an image whilst the breast remains compressed in said frame. This allows to position the breast in the second imaging device in the same position as in the first imaging device. Obviously in this arrangement the making of the first and second image can also be reversed.

In a possible embodiment the frame having the two plates is separable from the gamma radiation imaging apparatus and the frame is adapted to provide information and/or guidance for a procedure and/or treatment to be carried out on the object, e.g. a biopsy.

In a possible embodiment of the gamma radiation imaging apparatus according to the invention, a printer is provided that is adapted to print the image, preferably on an adhesive sticker, the sticker then being applied on the examined object, preferably the object previously being marked with the marker system and the sticker having one or more markers to be matched with the marking on the object so as to position the printed image correctly on the object.

In another embodiment the printed image is fitted on a plate of the separable frame of the gamma imaging apparatus, e.g. the printed image being adhesive. In a preferred embodiment, the plate is a piercable plate. As such, it may be possible to take a biopsy through the plate.

In another embodiment one or more electronic graphical displays are integrated or associated with one or more plates of the separable frame, e.g. positionable on top of a plate when the frame with plates is still on the object, yet removed from the imaging apparatus. For instance a plate may include or be combinable with an LCD-display, allowing to display at least one selected item from the reconstructed image.

In another embodiment the imaging device is associated with a projector, e.g. a color beamer device, the one or more plates being embodied as projection screens onto with the projector can display at least a selected item from the reconstructed image. Alignment of the projected image with the plate can be done electronically, e.g. as the projector senses the actual position of the plate acting as screen in order to align the projected image. Otherwise an alignment system can be associated with the projector to project the image in the correct position on the screen.

The one or more plates preferably have a thickness of less than 5 millimetres, more preferably between 1 and 3 millimetres.

The invention as described hereinabove will now be explained in more detail with reference to non-limiting exemplary embodiments, reference being made to the appended drawings, in which:
Figure 1 shows very diagrammatically an apparatus according to the invention,
Figure 2 very diagrammatically shows a detail of another embodiment of the apparatus according to the invention, and
Figure 3 very diagrammatically shows another embodiment of the apparatus according to the invention.

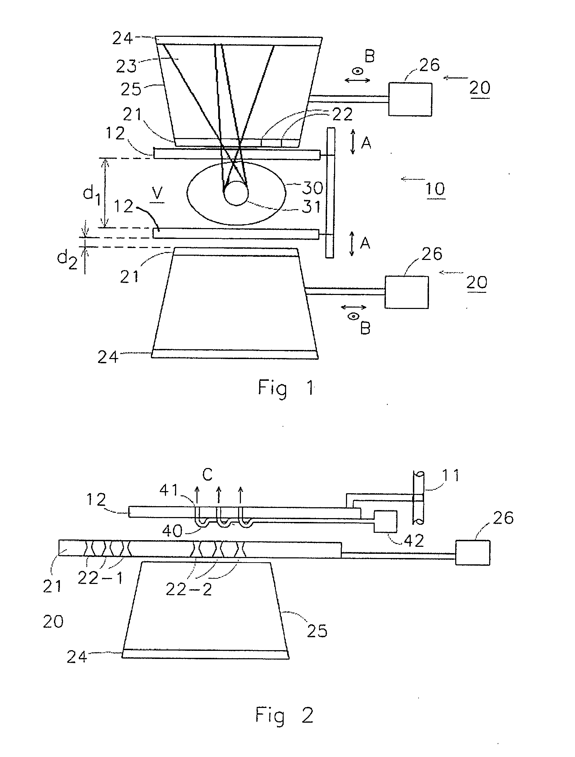

Figure 1 shows very diagrammatically an apparatus according to an embodiment of the invention, with an object positioning device 10 and two gamma cameras 20.

The object positioning device 10 as shown comprises a frame 11, with two plates 12, that are movable in the direction of arrows A, manually or by means of a plate mover means (not indicated). Note that only one plate need be adjustable, and that the frame may be dispensed with. The plates may be rotatably or slidably mounted to the frame 11, allowing the plates 12 to be moved to the side to open imaging space V. The plates 12 can be made of any sufficiently rigid, body compatible material, that is sufficiently transparent to gamma radiation, and preferably also to X-rays to allow use of an X-ray device. Furthermore, advantageously the material is optimally transparent to allow a visual check. Examples of useful materials are glass and plastics such as plexiglass or polycarbonate. The thickness could be as small as a few millimetres.

An imaging space V is defined between the plates 12, and its thickness is indicated by the parameter d1, which depends on the size of the object, e.g. a breast and on the degree of compression. In case only one plate 12 and one gamma camera 20 are provided, the space V is defined as the space on the side of the plate 12 facing away from the gamma camera 20, and having a thickness of between 1 and 20 cm.

In the imaging space V, a breast 30 is present, as the object to be imaged. As indicated, the breast 30 is not or hardly compressed, although shifting of the plates 12 may bring about a desired degree of compression, to decrease the thickness of the breast in perpendicular direction.

The gamma cameras 20 are taken to be of identical type, although in some embodiments this need not be the case, allowing e.g. different pinholes to be used. Here, they are identical, and each comprises a collimator plate 21 with pinholes 22, defining beams 23 that point toward a detector 24, all in a house 25, which is preferably but not necessarily radio-opaque. A collimator mover means is generally and diagrammatically indicated by 26. In this embodiment, the collimator mover means is able to move the house 25 with collimator 21 and detector 24 in the direction of the arrows B, pointing horizontally in and perpendicular to the plane of the paper. This is in particular beneficial when the camera is relatively lightweight, as future camera's will be. With the heavier and larger cameras as more common nowadays, it may be preferred to move only the collimator and keep the camera in position.

The collimator plate 21 comprises a plurality of pinholes 22. Each allows radiation emanating from the space V, from a volume in the shape of a cone, to pass and go to the detector 24. Two of these cones have been indicated in the drawing. The pinholes 22 are focused towards a focus volume 31, which is here a relatively small part of the space V. Such focused pinholes allow a lot of radiation and under many different angles to reach the detector 24, thus ensuring a high sensitivity and few artifacts. Since moreover the gamma camera 20 can be moved by means of the collimator mover means 26 in one or more of the directions B, even more data can be obtained, in that the volume 31 is scanned through the breast 30. Furthermore, the opening angle of the beams 23 may be selected by accordingly designing the pinholes 22. Additionally, and importantly, the absolute distance between the pinholes 22 and the volume 31, or generally e.g. the object 30, is very small. This allows a high magnification with very good resolution.

Note the distance d2 between the plate 12 and the collimator plate 21. d2 may be made very small, such as 3 cm or smaller. In practice, it suffices if the collimator plate 21 can move parallel to plate 12 without too much friction, so theoretically the distance d2 could be as small as substantially zero.

Figure 2 very diagrammatically shows a detail of another embodiment of the apparatus according to the invention. Herein, as in all of the drawings, similar parts are indicated by the same reference numerals, if necessary provided with a suffix such as "-1" and so on. Shown here is plate 12 with a marker system, as well as one gamma camera 20.

The plate 12 of the object positioning system is still connected to the frame 11, and now also has a marker system which comprises a plurality of channels 40 connected to openings 41, from which, in the direction of the arrows C, a marker substance can be provided by marker substance dispenser 42. The marker substance could be e.g. ink. Herein, printer technology could favorably be used, although other substances are not excluded. In use, dispenser 42 could provide marker substance to each of the openings 41, and create a grid on the breast, or could provide marker substance to one or more openings 41, to indicate corresponding regions of interest.

In use, the marker system may provide a visual indication of a region-of-interest, that could be subjected to further tests, such as a biopsy. It is also possible provide a reliable method of subsequent imaging. Thereto, it is advantageous to record a degree of compression of the breast, in that the distance d between the plates 12 is recorded during the first imaging (compare d1 Figure 1). Furthermore, the breast should be provided with one or more marks from the marking substance, with known positions corresponding to known openings 41. Then, in a subsequent imaging, the same compression, i.e. distance d, should be applied to the breast, while furthermore, the markings should be brought in register with the relevant openings 41 on the plate 21. This registering step is advantageously carried out before adjusting the distance. Then, as far as possible, the breast has assumed the same position with respect to the plate as it had in the first imaging session, and is now ready for registered second imaging.

Furthermore, the collimator plate 21 is shown with a first plurality of pinholes 22-1 and a second plurality of pinholes 22-2. The first pinholes 22-1 have a narrow opening angle, and could e.g. serve as a "zoom" setting, to view a smaller part of the image, while the second pinholes 22-2 have a larger opening angle, and could serve as a "wide angle view" setting, such as for an overview of the breast. The collimator mover means 26 could serve to shift the collimator plate such that the first pinholes are moved to the indicated position of the second pinholes. Note that it could also be one set of pinholes per collimator plate, with the collimator plates being exchangeable. Note furthermore that in this case the collimator plate is movable while the house 25 with the detector 24 is not moved, or could be independently movable with respect to the collimator plate 21.

As can be seen in the drawing, the actual pin-"hole", i.e. the most narrow constriction in the collimator plate 12, can be very close to the surface of the plate 12 that faces the imaging volume V. This is favorable for obtaining an even shorter distance to the object, and a favorable magnification factor.

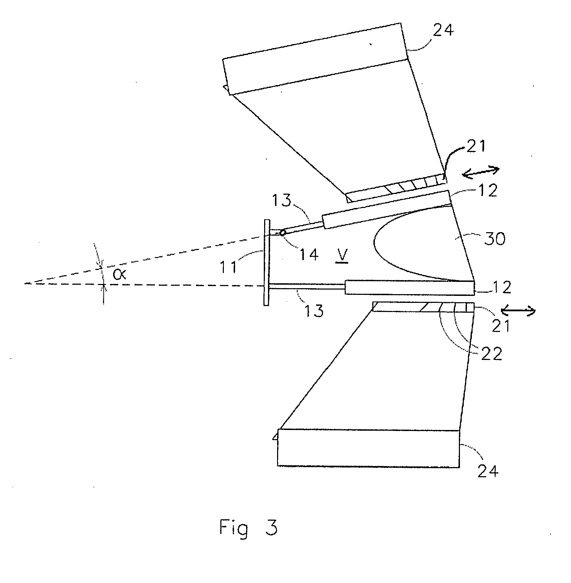

Figure 3 very diagrammatically shows another embodiment of the apparatus according to the invention, in a side elevational view.
Herein, a breast 30 is contacted by two plates 12, that are connected to the frame 11 by means of connections 13. One of the connections has e.g. a hinge or the like to position the two plates 12 in a V-shape, with a subtended angle a there between. In practice, the angle α can be selected such that the plates 12 contact the breast 30 efficiently and as comfortably as possible, i.e. by minimizing over compression, especially at the proximal end of the breast.

Note furthermore that the pinholes 21, indicated by the directions of their respective centerlines, are provided close to the proximal end of the breast, and/or that the centerlines of the pinholes are tilted towards said proximal end of the breast. This allows efficient scanning of as much breast tissue as possible. If the thickness of the breast tissue is locally larger, the scanning time may be increased accordingly.

Moreover, the collimators 21, which can again be moved in the directions of the arrows by means of non-shown collimator mover means, allow quick scans of the breast 30, thereby allowing a film or dynamic imaging of the breast, since in principle only the collimator 12 is moved, in one plane. It could be useful to provide an oversized detector 24 in such a case.

The specific embodiments shown here are to be understood merely as a non-limiting explanation of the invention, whose scope is defined by the appended claims.

## Claims

1. A gamma radiation imaging apparatus, comprising an object positioning device (10), defining an imaging space (V) for the object (30) to be imaged, and a gamma camera (20) positioned to image a volume in said imaging space (V), wherein the object positioning device (10) comprises two plates (12), with the imaging space (V) there between, wherein at least one of the plates (12) is movable in a direction substantially towards the other plate (12), and wherein the plates (12) are arranged to contact the object (30) when positioned between said plates (12),
and wherein the gamma camera (20) comprises a collimator (21) with at least one pinhole (22, 22-1, 22-2), and a gamma sensitive detector (24) arranged to receive images from the collimator (21), wherein the collimator (21) is arranged in a plane substantially parallel to one of the plates (12), **characterised in that** the collimator (21) is movable with respect to both said plates₋(12) in said plane, and the apparatus further comprises a collimator mover means (26) arranged to controllably move the collimator (21) in said plane.

2. Imaging apparatus according to claim 1, comprising a further gamma camera (20) which comprises a collimator (21) with at least one pinhole (22, 22-1, 22-2), and a gamma sensitive detector (24) arranged to receive images from the collimator (21), wherein the collimator (21) is arranged in a plane substantially parallel to one of the plates (12) and is preferably movable in said plane, and wherein the apparatus further preferably comprises a collimator mover means (26) arranged to controllably move the collimator (21) in said plane, and wherein the gamma camera (20) and the further gamma camera (20) are arranged on the opposite sides of the imaging space (V).

3. The apparatus of claim 1 or 2, wherein the apparatus comprises a frame (11) that supports the plates (12) in a mobile manner so as to be movable in a direction substantially towards one another allowing to compress the object (30) between the plates (12).

4. The apparatus of one or more of the preceding claims, wherein the gamma camera (20) and the collimator (21) are movable as a whole in a plane substantially parallel to the plate (12), and wherein the collimator mover means (26) is adapted to controllably move the gamma camera (20) and collimator (21) in said plane.

5. The apparatus of one or more of the preceding claims, wherein the apparatus has one or more collimators (21), thereby providing a plurality of different pinholes (22, 22-1, 22-2).

6. The apparatus of one or more of the preceding claims, wherein a collimator (21) has a plurality of pinholes (22, 22-1, 22-2), and wherein at least part of the plurality of pinholes (22, 22-1, 22-2) are focused towards a focus volume (31).

7. The apparatus of claim 6, wherein a first plurality of pinholes (22-1) are focused towards a first focus volume, and a second plurality of pinholes (22-2) are focused towards a second focus volume.

8. The apparatus of claim 6, wherein a first plurality of pinholes (22-1) has a first opening angle and a second plurality of pinholes (22-2) has a second opening angle, that is smaller than the first opening angle.

9. The apparatus of one or more of the preceding claims, wherein at least one pinhole (22, 22-1, 22-2) comprises a cross-slit pinhole, preferably wherein at least one cross-slit pinhole comprises adjustable crossed slits.

10. The apparatus of one or more of the preceding claims, further comprising a marker system (40, 41, 42), that is adapted to mark the object (30), preferably adapted to mark the object (30) while being positioned, preferably while being compressed, between the plates (12).

11. The apparatus of claim 10, wherein the marker system comprises a plurality of channels (40) in or on at least one of the plates (12) and opening out into ports (41) on the side of the imaging space (V), as well as a marker substance dispenser (42), arranged to controllably dispense marker substance through at least one of the channels (40).

12. The apparatus of one or more of the preceding claims, embodied as a gamma radiation breast imaging apparatus.

13. The apparatus of claim 3, wherein the frame (11) supporting the plates (12) is separable from the gamma radiation imaging apparatus.

14. A method of gamma imaging a breast (30), using an apparatus according to any preceding claim.

15. The method of claim 14, using an apparatus according to claims 10 and 12, comprising the steps of adjusting the plates (12) to a desired degree of compressing the breast (30), preferably recording distance d between the plates (12), imaging the breast (30), creating one or more marks on the breast (30) in known positions with respect to the plate (12) by means of the marker system (40, 41, 42), removing the breast (30) from the apparatus, and performing a second imaging, wherein the second imaging comprises registering the one or more marks on the breast (30) with respect to said known positions on the plate (12), adjusting the plates (12) to said distance d, and imaging the breast (30).

16. The method of claim 14, wherein the object positioning device comprises a separable frame (11) supporting the plates (12), such that the object (30) to be imaged may first be positioned in the frame (11), subsequently imaged by the gamma radiation imaging apparatus, and subsequently the frame (11) with the object (30) may be transferred to a second imaging apparatus.

## Patentansprüche

1. Gammastrahlungsbildgebungsvorrichtung umfassend eine Objektpositionierungsanordnung (10), die einen Bildgebungsbereich (V) für das zu erfassende Objekt (30) definiert, und eine Gammakamera (20), die derart positioniert ist, dass sie ein Volumen in dem Bildgebungsbereich (V) erfasst, wobei die Objektpositionierungsanordnung (10) zwei Platten (12) umfasst, zwischen denen der Bildgebungsbereich (V) liegt, wobei mindestens eine der Platten (12) im Wesentlichen in Richtung zu der anderen Platte (12) bewegbar ist, und wobei die Platten (12) derart angeordnet sind, dass sie das Objekt (30) berühren, wenn dieses zwischen den Platten (12) positioniert ist,
und wobei die Gammakamera (20) einen Kollimator (21) mit mindestens einer Lochblende (22, 22-1, 22-2) und einen gammasensitiven Detektor (24) umfasst, der derart angeordnet ist, dass er Bilder von dem Kollimator (21) empfängt, wobei der Kollimator (21) in einer Ebene angeordnet ist, die im Wesentlichen parallel zu einer der Platten (12) ist, **dadurch gekennzeichnet, dass** der Kollimator (21) in Relation zu beiden Platten (12) in der Ebene bewegbar ist, und wobei die Vorrichtung weiterhin eine Kollimatorbewegungseinrichtung (26) umfasst, die zum kontrollierten Bewegen des Kollimators (21) in der Ebene angeordnet ist.

2. Bildgebungsvorrichtung nach Anspruch 1, umfassend eine weitere Gammakamera (20), die einen Kollimator (21) mit mindestens einer Lochblende (22, 22-1, 22-2) und einen gammasensitiven Detektor (24) umfasst, der derart angeordnet ist, dass er Bilder von dem Kollimator (21) empfängt, wobei der Kollimator (21) in einer Ebene angeordnet ist, die im Wesentlichen parallel zu einer der Platten (12) ist, und vorzugsweise in der Ebene bewegbar ist, und wobei die Vorrichtung weiterhin vorzugsweise eine Kollimatorbewegungseinrichtung (26) umfasst, die zum kontrollierten Bewegen des Kollimators (21) in der Ebene angeordnet ist, und wobei die Gammakamera (20) und die weitere Gammakamera (20) an einander gegenüberliegenden Seiten des Bildgebungsbereichs (V) angeordnet sind.

3. Vorrichtung nach Anspruch 1 oder 2, wobei die Vorrichtung einen Rahmen (11) umfasst, der die Platten (12) in bewegbarer Art und Weise trägt, sodass sie im Wesentlichen in Richtung zueinander bewegbar sind und ein Einklemmen des Objekts (30) zwischen den Platten (12) ermöglichen.

4. Vorrichtung nach einem oder mehreren der vorhergehenden Ansprüche, wobei die Gammakamera (20) und der Kollimator (21) als Ganzes in einer Ebene bewegbar sind, die im Wesentlichen parallel zu der Platte (12) ist, und wobei die Kollimatorbewegungseinrichtung (26) zum kontrollierten Bewegen der Gammakamera (20) und des Kollimators (21) in der Ebene ausgebildet ist.

5. Vorrichtung nach einem oder mehreren der vorhergehenden Ansprüche, wobei die Vorrichtung einen oder mehrere Kollimatoren (21) und somit eine Vielzahl von unterschiedlichen Lochblenden (22, 22-1, 22-2) aufweist.

6. Vorrichtung nach einem oder mehreren der vorhergehenden Ansprüche, wobei ein Kollimator (21) eine Vielzahl von Lochblenden (22, 22-1, 22-2) aufweist, und wobei mindestens ein Teil der Vielzahl von Lochblenden (22, 22-1, 22-2) auf ein Fokussierungsvolumen (31) fokussiert ist.

7. Vorrichtung nach Anspruch 6, wobei eine erste Vielzahl von Lochblenden (22-1) auf ein erstes Fokussierungsvolumen fokussiert ist, und eine zweite Vielzahl von Lochblenden (22-2) auf ein zweites Fokussierungsvolumen fokussiert ist.

8. Vorrichtung nach Anspruch 6, wobei eine erste Vielzahl von Lochblenden (22-1) einen ersten Öffnungswinkel aufweist, und eine zweite Vielzahl von Lochblenden (22-2) einen zweiten Öffnungswinkel aufweist, der kleiner als der erste Öffnungswinkel ist.

9. Vorrichtung nach einem oder mehreren der vorhergehenden Ansprüche, wobei mindestens eine Lochblende (22, 22-1, 22-2) eine Kreuzschlitzlochblende umfasst, wobei vorzugsweise mindestens eine Kreuzschlitzlochblende verstellbare Kreuzschlitze umfasst.

10. Vorrichtung nach einem oder mehreren der vorhergehenden Ansprüche, weiterhin umfassend ein Markierungssystem (40, 41, 42), das ausgebildet ist, das Objekt (30) zu markieren, vorzugsweise ausgebildet, das Objekt (30) während der Positionierung zu markieren, vorzugsweise während des Einklemmens zwischen den Platten (12).

11. Vorrichtung nach Anspruch 10, wobei das Markierungssystem eine Vielzahl von Kanälen (40) in oder auf mindestens einer der Platten (12), die auf der Seite des Bildgebungsbereichs (V) in Öffnungen (41) enden, sowie eine Markierungssubstanzabgabeeinheit (42), die zur kontrollierten Abgabe einer Markierungssubstanz durch mindestens einen der Kanäle (40) angeordnet ist, umfasst.

12. Vorrichtung nach einem oder mehreren der vorhergehenden Ansprüche, in der Ausführungsform einer Gammastrahlungs-Brustbildgebungsvorrichtung.

13. Vorrichtung nach Anspruch 3, wobei der Rahmen (11), der die Platten (12) trägt, von der Gammastrahlungsbildgebungsvorrichtung trennbar ist.

14. Verfahren für eine Gammastrahlungsbildgebung einer Brust (30), wobei eine Vorrichtung nach einem der vorhergehenden Ansprüche verwendet wird.

15. Verfahren nach Anspruch 14, wobei eine Vorrichtung nach Anspruch 10 und 12 verwendet wird, umfassend die Schritte eines Einstellens der Platten (12) auf einen gewünschten Grad eines Einklemmens der Brust (30), wobei vorzugsweise ein Abstand d zwischen den Platten (12) aufgezeichnet wird, einer Bildgebung der Brust (30), wobei eine oder mehrere Markierungen auf der Brust (30) an bekannten Positionen in Relation zu der Platte (12) durch das Markierungssystem (40, 41, 42) erzeugt werden, eines Entfernens der Brust (30) aus der Vorrichtung, und eines Durchführens einer zweiten Bildgebung, wobei die zweite Bildgebung ein Registrieren der einen oder mehreren Markierungen auf der Brust (30) in Relation zu den bekannten Positionen auf der Platte (12), ein Einstellen der Platten (12) auf den Abstand d, und eine Bildgebung der Brust (30) umfasst.

16. Verfahren nach Anspruch 14, wobei die Objektpositionierungsanordnung einen abtrennbaren Rahmen (11) umfasst, der die Platten (12) derart trägt, dass das zu erfassende Objekt (30) zuerst in dem Rahmen (11) positioniert werden kann, anschließend eine Bildgebung durch die Gammastrahlungsbildgebungsvorrichtung erfolgen kann, und anschließend der Rahmen (11) mit dem Objekt (30) zu einer zweiten Bildgebungsvorrichtung übertragen werden kann.

## Revendications

1. Appareil d'imagerie à rayonnement gamma, comprenant un dispositif de positionnement d'objet (10), définissant un espace d'imagerie (V) pour l'objet (30) dont l'image doit être formée, et une gamma caméra (20) positionnée pour former l'image d'un volume dans ledit espace d'imagerie (V), dans lequel le dispositif de positionnement d'objet (10) comprend deux plaques (12), l'espace d'imagerie (V) étant entre elles, dans lequel au moins l'une des plaques (12) peut être déplacée dans une direction sensiblement vers l'autre plaque (12), et dans lequel les plaques (12) sont agencées pour venir en contact avec l'objet (30) lorsqu'il est positionné entre lesdites plaques (12),
et dans lequel la gamma caméra (20) comprend un collimateur (21) avec au moins un trou sténopéique (22, 22-1, 22-2), et un détecteur sensible au rayonnement gamma (24) agencé pour recevoir des images du collimateur (21), dans lequel le collimateur (21) est agencé dans un plan sensiblement parallèle à l'une des plaques (12), **caractérisé en ce que** le collimateur (21) peut être déplacé par rapport aux dites deux plaques (12) dans ledit plan, et l'appareil comprend en outre des moyens de déplacement de collimateur (26) agencés pour déplacer le collimateur (21) de manière contrôlable dans ledit plan.

2. Appareil d'imagerie selon la revendication 1, comprenant une gamma caméra (20) supplémentaire qui comprend un collimateur (21) avec au moins un trou sténopéique (22, 22-1, 22-2), et un détecteur sensible au rayonnement gamma (24) agencé pour recevoir des images du collimateur (21), dans lequel le collimateur (21) est agencé dans un plan sensiblement parallèle à l'une des plaques (12) et peut être déplacé de préférence dans ledit plan, et dans lequel l'appareil comprend en outre de préférence des moyens de déplacement de collimateur (26) agencés pour déplacer le collimateur (21) de manière contrôlable dans ledit plan, et dans lequel la gamma caméra (20) et la gamma caméra (20) supplémentaire sont agencées sur les côtés opposés de l'espace d'imagerie (V).

3. Appareil selon la revendication 1 ou 2, dans lequel l'appareil comprend un cadre (11) qui supporte les plaques (12) d'une manière mobile de façon à ce qu'elles puissent être déplacées sensiblement dans une direction l'une vers l'autre, ce qui permet la compression de l'objet (30) entre les plaques (12).

4. Appareil selon une ou plusieurs des revendications précédentes, dans lequel la gamma caméra (20) et le collimateur (21) peuvent être déplacés ensemble dans un plan sensiblement parallèle à la plaque (12), et dans lequel les moyens de déplacement de collimateur (26) sont conçus pour déplacer la gamma caméra (20) et le collimateur (21) de manière contrôlable dans ledit plan.

5. Appareil selon une ou plusieurs des revendications précédentes, dans lequel l'appareil comporte un ou plusieurs collimateurs (21), fournissant de ce fait une pluralité de trous sténopéiques (22, 22-1, 22-2) différents.

6. Appareil selon une ou plusieurs des revendications précédentes, dans lequel un collimateur (21) comporte une pluralité de trous sténopéiques (22, 22-1, 22-2), et dans lequel au moins une partie de la pluralité de trous sténopéiques (22, 22-1, 22-2) sont focalisés vers un volume de focalisation (31).

7. Appareil selon la revendication 6, dans lequel une première pluralité de trous sténopéiques (22-1) sont focalisés vers un premier volume de focalisation, et une deuxième pluralité de trous sténopéiques (22-2) sont focalisés vers un deuxième volume de focalisation.

8. Appareil selon la revendication 6, dans lequel une première pluralité de trous sténopéiques (22-1) ont un premier angle d'ouverture et une deuxième pluralité de trous sténopéiques (22-2) ont un deuxième angle d'ouverture, qui est plus petit que le premier angle d'ouverture.

9. Appareil selon une ou plusieurs des revendications précédentes, dans lequel au moins un trou sténopéique (22, 22-1, 22-2) comprend un trou sténopéique à fentes croisées, de préférence dans lequel au moins un trou sténopéique à fentes croisées comprend des fentes croisées ajustables.

10. Appareil selon une ou plusieurs des revendications précédentes, comprenant en outre un système de marquage (40, 41, 42), qui est conçu pour marquer l'objet (30), de préférence conçu pour marquer l'objet (30) alors qu'il est positionné, de préférence alors qu'il est comprimé, entre les plaques (12).

11. Appareil selon la revendication 10, dans lequel le système de marquage comprend une pluralité de canaux (40) dans ou sur au moins l'une des plaques (12) et s'ouvrant en des orifices (41) du côté de l'espace d'imagerie (V), ainsi qu'un distributeur de substance de marquage (42), agencé pour distribuer une substance de marquage de manière contrôlable à travers au moins l'un des canaux (40).

12. Appareil selon une ou plusieurs des revendications précédentes, mis en oeuvre en tant qu'appareil d'imagerie du sein à rayonnement gamma.

13. Appareil selon la revendication 3, dans lequel le cadre (11) supportant les plaques (12) peut être séparé de l'appareil d'imagerie à rayonnement gamma.

14. Procédé d'imagerie par rayonnement gamma d'un sein (30), utilisant un appareil selon l'une quelconque des revendications précédentes.

15. Procédé selon la revendication 14, utilisant un appareil selon les revendications 10 et 12, comprenant les étapes d'ajustement des plaques (12) à un degré souhaité de compression du sein (30), d'enregistrement de préférence d'une distance d entre les plaques (12), d'imagerie du sein (30), de création d'une ou de plusieurs marques sur le sein (30) à des positions connues par rapport à la plaque (12) au moyen du système de marquage (40, 41, 42), de retrait du sein (30) de l'appareil, et d'exécution d'une deuxième imagerie, dans lequel la deuxième imagerie comprend l'alignement desdites une ou plusieurs marques sur le sein (30) avec lesdites positions connues sur la plaque (12), l'ajustement des plaques (12) à ladite distance d, et l'imagerie du sein (30).

16. Procédé selon la revendication 14, dans lequel le dispositif de positionnement d'objet comprend un cadre (11) séparable supportant les plaques (12), de sorte que l'objet (30) dont l'image doit être formée puisse d'abord être positionné dans le cadre (11), que l'image puisse être formée par la suite par l'appareil d'imagerie à rayonnement gamma, et que le cadre (11) avec l'objet (30) puisse ensuite être transféré à un deuxième appareil d'imagerie.
